# EUROPEAN PATENT SPECIFICATION

(11) **EP 1 741 431 B1**
(45) Date of publication and mention of the grant of the patent: **15.08.2012**
(21) Application number: 05752335.9
(22) Date of filing: 29.04.2005
(51) Int. Cl.: A61K 31/4965, A61K 31/40, A61P 3/06, A61K 45/06

(54) **THE COMBINATION FOR TREATING HYPERLIPEMIA**
KOMBINATION ZUR BEHANDLUNG VON HYPERLIPIDÄMIE
COMBINAISON POUR LE TRAITEMENT DE L'HYPERLIPIDEMIE

(30) Priority: 30.04.2004 CN 200410024066
(43) Date of publication of application: 10.01.2007
(73) Proprietor: Lunan Pharmaceutical Group Corporation, Linyi, Shandong 276006 (CN)
(72) Inventor: ZHAO, Zhiquan, Shandong 276005 (CN)
(74) Representative: Gillet, Raphaëlle
(86) International application number: PCT/CN2005/000608
(87) International publication number: WO 2005/105095

(56) References cited:
- WO-A-01/28999
- WO-A-02/058685
- CN-A- 1 425 374
- CN-A- 1 486 695
- US-A- 4 002 750
- US-A- 5 260 305
- US-A1- 2003 162 827

## Description

### FIELD OF THE INVENTION

The present invention relates to a novel composition for treating hyperlipaemia, comprising the first active component acipimox, and the second active component atorvastatin, or the pharmaceutically acceptable salt, ester or solvate thereof.

### BACKGROUND OF THE INVENTION

With the continuous development of the medical sciences, it is recognized that the excessive amount of cholesterol, lipid and the like is the basic cause of cardiovascular diseases, and high level of blood lipid is the main dangerous factor related to the occurrence of coronary heart disease and hypertension. Therefore, the development of blood lipid modulating agents is considered as the focus of preventing and treating cardiovascular diseases. Since the end of 1980s, a large amount of agents for lowering blood lipid have been developed, among which statins are well estimated, because the excellent clinical therapeutic effect of statins is much higher than other blood lipid modulating agents. During more than ten years, the results of several international large scale trials on preventing and treating coronary heart disease demonstrated that statins can decrease the morbidity and mortality rates of coronary heart disease, and slower the progression of the already developed atherosclerotic clot, and even reduce the atherosclerotic clot, thereby broke the traditional concept which says that coronary heart disease cannot be reversed. A revolution of blood lipid initiated by "statins" is springing up all over the world. Currently, people in the medical art are full of confidence on the effects of lipid modulating agents for preventing and treating cardiovascular diseases, and the lipid modulating therapy will be the main method for preventing and treating cardiovascular diseases in the 21 century.

Atorvastatin is a lipid modulating agent, which is the third generation of statins. It is a completely synthesized inhibitor of hydroxymethylglutaryl-CoA (HMG-CoA) reducase, firstly launched into the market of USA in the form of calcium salt in 1995. Its chemical name is: calcium [R-(R*,R*)]2-(4-fluorophenyl)-βδ-dihydroxyl-5-(1-methylethyl)-3-phenyl-4-[aniline]carbonyl]-1-hydro-pyrrolyl-1-heptanoate trihydrate. 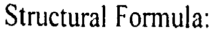 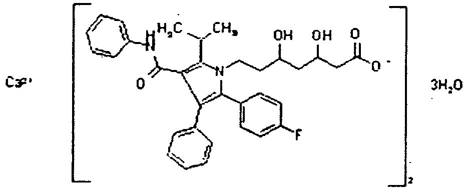 Molecular formula: (C₃₃H₃₄ FN₂O₅)₂Ca·3H₂O

Generally said atorvastatin refers to the above-mentioned trihydrate of calcium salt, which is also known as atorvastatin calcium, and the dosages/specifications are generally calculated based on atorvastatin free acid. Chinese Daoyi Website (www.daoyi.com/drug/html/200009/6000000189670.html) reviews its pharmacodynamics as following:
1. Inhibiting HMG-CoA reducase. HMG-CoA reducase is a rate-limiting enzyme in the enzyme systems for synthesizing cholesterol, the inhibition of which will cause the synthesis of cholesterol to be decreased. Atorvastatin shows biological activity immediately after the absorption by oral administration as pravastatin, while both of lovastatin and simvastatin are prodrugs, which show biological activity only after being metabolized *in vivo.* Therefore, the effect of atorvastatin is faster than those of lovastatin and simvastatin. The inhibitory effect of atorvastatin on HMG-CoA reducase is higher than those of other statins. 50% Inhibition concentration (IC50) of atorvastatin on HMG-CoA reducase is 73 nmol.L⁻¹, while that of pravastatin is 2650 nmol.L⁻¹. Atorvastatin 80 mg/d, pravastatin 40 mg/d and simvastatin 40 mg/d are shown to reduce the plasma concentration of methyl dihydroxyl valeric acid (MVA) by 59%, 32% and 49%, respectively. Moreover, the inhibiting duration of single dosage of atorvastatin on HMG-CoA reducase is longer than those of other statins.
2. Increasing the receptor of low density lipoprotein (LDL). By the inhibition of HMG-CoA reducase, atorvastatin decreases the concentration of cholesterol in plasma and tissue cells, and stimulates the increase of the density of LDL receptor in the liver, thereby enhance the clearance of plasma low density lipoprotein-cholesterol (LDL-C). LDL receptor in the liver may bind to very low density lipoprotein (VLDL) to enhance the degradation of VLDL, thereby decrease the concentration of triglyceride (TG).
3. Inhibiting the synthesis of very low density lipoprotein-cholesterol (VLDL-C). Cholesterol is required for synthesizing VLDL. Atorvastatin may decrease the synthesis and secretion of VLDL by decreasing the plasma concentration of cholesterol. VLDL is required for carrying and transporting TG, and VLDL-C is the precursor of LDL-C, thus atorvastatin may decrease the level of TG, VLDL-C and LDL-C.
4. Anti-atherosclerotic effect. Atorvastatin is advantageous to delay the progression of atherosclerosis by decreasing the blood lipid, reducing the lipid infiltration and the formation of foam cells. Atorvastatin also may prevent atherosclerotic clot from breaking. Furthermore, *in vitro* experiments show that atorvastatin may inhibit the proliferation and migration of vascular smooth muscles. *In vivo* experiments show that 2.5 mg/kg of atorvastatin may decrease the area of rabbit atherosclerotic clot by 67% (P<0.05), while lovastatin, pravastatin and simvastatin at the same dosage do not show such effect. Atorvastatin also has the effects of activating platelet, decreasing blood viscosity, inhibiting coagulation, and the like. The clinical studies show that the administration of atorvastatin 80 mg/d to 22 hyperlipemia patients decreases the plasma viscosity by 10%, decreases the activity of coagulation factor VII by 8%, and decreases the coagulation rate of platelet induced by thromboxane A2 (TXA2) by 11%. All these effects are advantageous to prevent atherosclerosis.

Generally, atorvastatin is well tolerated, and most of adverse effects are slight. The tolerance of patient to atorvastatin is better than lovastatin.

Acipimox is an artificially synthesized nicotinic acid derivative, which can inhibit the decomposition of fat tissue, decrease the release of free fat acid from fat tissue, thereby reducing the synthesis of triglyceride (TG) in liver. Furthermore, Acipimox may decrease the concentration of triglyceride (TG) and total cholesterol (TC) in serum by inhibiting the synthesis of very low density lipoprotein (VLDL) and low density lipoprotein (LDL). Acipimox may also inhibit the activity of lipase in the liver, and decrease the decomposition of high density lipoprotein (HDL). Acipimox is quickly absorbed after oral administration, and the peak of plasma concentration can be reached within 2 hours after administration, with the half life of 2 hours. Acipimox does not combine with plasma protein, and is excreted mainly in the form of Acipimox itself, without being metabolized. In clinical application, Acipimox can effectively treat hypertriglyceridemia (type IV), hypercholesterolemia (type IIa) and hypertriglyceridemia plus hypercholesterolemia (type IIb). Acipimox is a safe, effective, and a well tolerated blood lipid modulating agent.

Currently, the research tendency in this art is to formulate two blood lipid modulating agents taking actions in different action mechanisms into a combined preparation, and thereby allow the lipid decreased effect more extensive, and also allow them act synergistically to enhance the therapeutic effect and reduce adverse effects.

US patent No. US5260305A discloses a composition comprising pravastatin, an inhibitor of HMG-CoA reducase, and nicotinic acid as well as the derivative thereof, and particularly discloses the preparation of compositions comprising 5 mg, 10 mg, 20 mg and 40 mg of pravastatin respectively and 750 mg of acipimox. However, the patent does not disclose the beneficial effect thereof and the pharmacological experimental data of the best formulation.

Chinese application publication No. CN1425374A discloses a composition comprising acipimox and lovastatin, in which the weight ratio of acipimox to lovastatin is 25-50:1, preferably 25:1 or 37.5:1. However, the patent does not mention the composition comprising acipimox and atorvastatin, particularly the best formulation of the composition, and the corresponding pharmacologically experimental data thereof.

### SUMMARY OF THE INVENTION

The object of the present invention is to provide a novel pharmaceutical composition for treating hyperlipemia, comprising the first active component acipimox, and the second active component atorvastatin, or the pharmaceutically acceptable salt, ester or solvate thereof. The resultant composition has more extensive lipid decreasing effect due to the different action mechanisms of acipimox and atorvastatin. Moreover, the two components can act synergistically, and the lipid decreasing effect is superior to either of the two components used alone at the same dosage. Furthermore, by properly selecting the amount of atorvastatin in the composition, not only may the composition effectively decrease the blood lipid level but also may show no obvious toxic adverse effect. Meanwhile, the composition may be conveniently administered only once per day, thereby greatly increase the compliance of the patients. Furthermore, by comparing the composition according to the present invention with the compositions in the prior art US5260305A and CN1425374A, the inventors found unexpectedly that the combination of acipimox and atorvastatin shows remarkably synergistic effect in decreasing serum total cholesterol, serum triglyceride and serum low density lipoprotein-cholesterol. Furthermore, in comparison with the combination of acipimox and pravastatin or the combination of acipimox and lorvastatin, the combination of acipimox and atorvastatin shows more remarkable lipid decreasing effect and high density lipoprotein increasing effect.

One of the compositions according to the present invention consists of acipimox and atorvastatin calcium, as well as pharmaceutically acceptable adjuvants, in which the weight ratio of acipimox to atorvastatin calcium (calculated by the free acid, the same below) is 10-80:1, preferably 10-30:1, more preferably 30:1. The dosage form of the pharmaceutical composition includes solid formulations, such as tablets, capsules, granules, pills, dropping pills and the like, which can prepared by the general preparing methods well known in the art. The content of acipimox corresponds to a daily dosage of about 200 to 750 mg, and the content of atorvastatin calcium corresponds to a daily dosage of 5 to 80 mg, preferably 10 to 30 mg, when administrating the composition.

Upon formulating the composition according to the present invention into solid formulations such as tablets or capsules, in order to achieve a permanent therapeutic effect, effective amount of acipimox is preferably formulated into a slow released part, and then formulated into slow released formulations such as slow released tablets or slow released capsules and the like together with effective amount of atorvastatin calcium. Accordingly, pharmaceutically acceptable adjuvants include a diluent, such as starch, lactose, mannitol, pregelatinized starch, dextrine, microcrystal cellulose; a disintegrant, such as carboxymethyl starch sodium, hydroxypropyl starch, low substituted hydroxypropyl cellulose, carboxymethylcellulose sodium; slow-release agent, such as ethylcellulose, hydroxypropyl methylcellulose-4M, hydroxypropyl methylcellulose-15M, Eudragit RS-100, RL100, RS30D, RL30D, NE30D, as well as Surelease (water dispersion of ethylcellulose); an adhesive, such as polyvinylpyrrolidone, crosslinked polyvinylpyrrolidone, etc.; a lubricant, such as magnesium stearate, talc powder, micronized silica gel, and the like.

Through the pharmacological researches, it is shown that in comparison with effective amount of acipimox or atorvastatin calcium alone, the composition according to the present invention, especially in a preferred ratio, shows surprisingly better effect, without increasing any toxicity. Therefore, the safe dosage range of the composition according to the present invention is broad, the duration of therapeutic effect is long, and thus comprehensive effect is favorable, and is further convenient for administration. The composition according to the present invention may be administered once or twice per day, preferably once per day. In comparison with the combination of acipimox and pravastatin, and the combination of acipimox and lovastatin, the composition according to the present invention also shows better lipid decreasing activity.

In addition, we found from the screening experiments that the pharmacological activity of atorvastatin in the composition according to the present invention had little relationship with the salt, ester or solvate thereof. The lipid decreasing activity only relates to the amount of atorvastatin free acid. Therefore, atorvastatin in the present invention may be any one of the pharmaceutically acceptable salts, ie., any one of the appropriate physiologically acceptable salts of atorvastatin, including salts formed with inorganic base and organic base, such as sodium salt, calcium salt, potassium salt, magnesium salt, zinc salt and iron salt; or any one of the pharmaceutically acceptable esters of atorvastatin, ie., any one of the appropriate physiologically acceptable esters, including esters formed with aliphatic alcohol, aromatic alcohol and heterocylic alcohol, such as methyl ester, ethyl ester, allyl ester, and phenyl ester.

### DETAILED DESCRIPTION OF THE INVENTION

The composition according to the present invention and the method for preparing the same as well as the therapeutical effect thereof will be further described by the following examples. However, the scope of the present invention is not limited to the examples.

### Example 1

| | | |
|---|---|---|
| a. | Acipimox | 200g |
| | Blank pill core | 250g |
| | 7% PVP solution (90% ethanol as solvent) | 200g |

Preparing process: Passing Acipimox through a sieve of 120 mesh, weighing acipimox as shown in the formulation, and then the weighed acipimox was poured into a feeding hopper. Switching on a granulating and coating machine, with the pressure of inward wind 0.5 bar, the temperature of inward wind 30°C. the pressure of the spray gun (CYL) 3 bar, atomizing pressure (CAP1) 0.8 bar, and pouring the blank pill core for granulating, then the granulation is conducted under the condition of feeding rate 4 rpm, peristaltic pump 12%, rotating rate of the turnplate 145 rpm, and spraying with 7% PVP solution (90% ethanol as solvent). After granulating, the resultant granules were dried at 50°C, and then discharged.

| | | |
|---|---|---|
| b. | Atorvastatin calcium | 5g (calculated by the free acid) |
| | Blank pill core | 30g |
| | 7% PVP solution (90% ethanol as solvent) | 30g |

Preparing process: Passing atorvastatin calcium through a sieve of 120 mesh, weighing atorvastatin calcium as shown in the formulation, and then the weighed atorvastatin calcium was poured into a feeding hopper. Switching on granulating and coating machine, with the pressure of inward wind 0.5 bar, the temperature of inward wind 30°C, the pressure of the spray gun (CYL) 3 bar, atomizing pressure (CAP1) 0.8 bar, and pouring the blank pill core in the amount as shown in the formulation, and then the granulation was conducted under the condition of feeding rate 4 rpm, peristaltic pump 6%, rotating rate of the turnplate 160 rpm, and spraying with 7% PVP solution (90% ethanol as solvent). After granulating, the resultant granules were dried at 45°C, and then were discharged.
c. Filling the pellets prepared above in a and b into hard capsules by using a filling machine, with 200 mg of acipimox and 5 mg of atorvastatin calcium (calculated by the free acid, i.e., it is actually 5mg of atorvastatin free acid) contained in every two capsules.

### Example 2

| | | |
|---|---|---|
| a. | Acipimox | 200g |
| | Lactose | 30g |
| | Carboxymethyl starch sodium | 30g |
| | Microcrystal cellulose | 18g |
| | 6% PVP in anhydrous ethanol | 100g |
| | Magnesium stearate | 2g |

Preparing process: Passing acipimox through a sieve of 100 mesh, and passing lactose, carboxymethyl starch sodium and microcrystal cellulose through a sieve of 80 mesh; weighing acipimox, lactose, carboxymethylstarch sodium and microcrystal cellulose as shown in the formulation, and then mixing them uniformly; and appropriate amount of 6% PVP in anhydrous ethanol was added into the mixture for granulating. After granulating, drying the granules at 60°C, and then passing the granules through a sieve of 16 mesh; magnesium stearate with the weight shown in the formulation was then mixed into the dry granules.

| | | |
|---|---|---|
| b. | Atorvastatin calcium | 10g (calculated by the free acid) |
| | Hydroxypropyl cellulose | 15g |
| | Pregelatinized starch | 10g |
| | 6% PVP in anhydrous ethanol | 30g |
| | Glyceryl behenate | 1g |

Preparing process: Passing atorvastatin calcium through a sieve of 100 mesh, and passing hydroxypropyl cellulose and pregelatinized starch through a sieve of 80 mesh, weighing them as shown in the formulation, and then mixing them uniformly, after that an appropriate amount of 6% PVP in anhydrous ethanol was added into the mixture for granulating. After granulating, drying the granules at 60°C, and then passing the granules through a sieve of 16 mesh, glyceryl behenate with the weight shown in the formulation was then mixed into the dry granules.
c. The resultant granules in a and b were compressed into double layer tablets by using double punches compression machine, with 200mg of acipimox and 10mg of atorvastatin calcium (calculated by the free acid) contained in each tablet.

### Example 3

| | | |
|---|---|---|
| a. | Acipimox | 200g |
| | Lactose | 30g |
| | Carboxymethylstarch sodium | 30g |
| | Microcrystal cellulose | 18g |
| | 6% PVP in anhydrous ethanol | 100g |
| | Magnesium stearate | 2g |

Preparing process: Passing acipimox through a sieve of 100 mesh, and passing lactose, carboxymethylstarch sodium and microcrystal cellulose through a sieve of 80 mesh; weighing them as shown in the formulation, and then mixing them uniformly; and then appropriate amount of 6% PVP in anhydrous ethanol was added into the mixture for granulating. After granulating, drying the granules at 60°C, and then passing the granules through a sieve of 16 mesh; magnesium stearate with the weight shown in the formulation was then mixed into the dry granules.

| | | |
|---|---|---|
| b. | Atorvastatin calcium | 20g (calculated by the free acid) |
| | hydroxypropyl cellulose | 30g |
| | Pregelatinized starch | 20g |
| | 6% PVP in anhydrous ethanol | 50g |
| | Glyceryl behenate | 2g |

Preparing process: Passing atorvastatin calcium through a sieve of 100 mesh, and passing hydroxypropyl cellulose and pregelatinized starch through a sieve of 80 mesh, and weighing them as shown in the formulation, and then mixing them uniformly, after that an appropriate amount of 6% PVP in anhydrous ethanol was added into the mixture for granulating. After granulating, drying the granules at 60°C, and then passing the granules through a sieve of 16 mesh, glyceryl behenate with the weight shown in the formulation was then mixed into the dry granules.
c. The resultant granules in a and b were compressed into double layer tablets by using double punches compression machine, with 200mg of acipimox and 20mg of atorvastatin calcium (calculated by the free acid) contained in each tablet.

### Example 4

| | | |
|---|---|---|
| a. | Acipimox | 300g |
| | Hydroxypropyl methylcellulose-4M | 40g |
| | Microcrystal cellulose | 30g |
| | 8% PVP in anhydrous ethanol | 150g |
| | Magnesium stearate | 2g |

Preparing process: Passing acipimox through a sieve of 100 mesh, and passing hydroxypropyl methylcellulose-4M and microcrystal cellulose through a sieve of 80 mesh, and weighing them as shown in the formulation, and then mixing them uniformly, and then appropriate amount of 8% PVP in anhydrous ethanol was added into the mixture for granulating. After granulating, drying the granules at 60°C, and then passing the granules through a sieve of 16 mesh, magnesium stearate with the weight shown in the formulation was then mixed into the dry granules.

| | |
|---|---|
| b. Atorvastatin calcium | 20g (calculated by the free acid) |
| carboxymethylcellulose sodium | 30g |
| Lactose | 20g |
| 6% PVP in 95% ethanol solution | 50g |
| Magnesium stearate | 2g |

Preparing process: Passing atorvastatin calcium through a sieve of 100 mesh, and passing lactose and carboxymethylcellulose sodium through a sieve of 80 mesh, and weighing them as shown in the formulation, and then mixing them uniformly, after that an appropriate amount of 6% PVP in 95% ethanol solution was added into the mixture for granulating. After granulating, drying the granules at 60°C, and then passing the granules through a sieve of 16 mesh, magnesium stearate with the weight shown in the formulation was then mixed into the dry granules.
c. The resultant granules in a and b were compressed into double layer tablets by using double punches compression machine, with 300mg of acipimox and 20mg of atorvastatin calcium (calculated by the free acid) contained in each tablet.

### Example 5

| | | |
|---|---|---|
| a. | Acipimox | 300g |
| | Blank pill core | 250g |
| | 7% PVP solution (90% ethanol as solvent) | 200g |

Preparing process: Passing Acipimox through a sieve of 120 mesh, weighing acipimox as shown in the formulation, and then the acipimox were poured into a feeding hopper. Switching on a granulating and coating machine, with the pressure of inward wind 0.5 bar, the temperature of inward wind 30°C, CYL: 3 bar, CAP1: 0.8 bar, and pouring the blank pill core with the weight as shown in the formulation, and then granulation was conducted under the condition of feeding rate 4 rpm, peristaltic pump 12%, rotating rate of the turnplate 145 rpm, while spraying 7% PVP solution (90% ethanol as solvent). After granulating, the resultant granules were dried at 50°C, and then were discharged.

| | | |
|---|---|---|
| b. | pellets comprising acipimox prepared in a Surelease | 90g |
| | Talc powder | 1g |
| | Pure water | 50g |

Preparing process: Pellets comprising acipimox prepared in "a" were poured into the turnplate. Switching on granulating and coating machine, with the pressure of inward wind 1.0 bar, the temperature of inward wind 30°C, CYL: 3 bar, CAP1: 1.5 bar, peristaltic pump 5%, rotating rate of the turnplate 180 rpm, and then Surelease aqueous solution was sprayed into machine. After coating, the resultant materials were dried at 50°C, and then were discharged.
c. The atorvastatin calcium pellets were prepared as the method described in b of Example 1, and then filled into hard capsules by using a capsule filling machine together with the acipimox pellets prepared in b of the present Example, with 300 mg of acipimox and 10 mg of atorvastatin calcium (calculated by the free acid) contained in every two capsules.

### Example 6

| | | |
|---|---|---|
| a. | Acipimox | 300g |
| | Blank pill core | 300g |
| | 7% PVP solution (90% ethanol as solvent) | 200g |

Preparing process: Passing Acipimox through a sieve of 120 mesh, weighing acipimox as shown in the formulation, and then the weighed acipimox were poured into a feeding hopper. Switching on granulating and coating machine, with the pressure of inward wind 0.5 bar, the temperature of inward wind 30°C, CYL: 3 bar, CAP1: 0.8 bar, and pouring the blank pill core, and then granulating under the condition of feeding rate 4 rpm, peristaltic pump 12% and rotating rate of the turnplate 145 rpm, and then 7% PVP solution (90% ethanol as solvent) was sprayed into the material in the machine. After granulating, drying the resultant granules at 50°C, and then the dry granules were discharged.
b. pellets comprising acipimox prepared in a

| | | |
|---|---|---|
| | Ethylcellulose | 40g |
| | Stearic acid | 70g |
| | Polyethylene glycol-6000 | 6g |
| | Talc powder | 12g |
| | 95% ethanol | 1000g |

Preparing process: Pellets comprising acipimox prepared in "a" were poured into a feeding hopper. Switching on granulating and coating machine, with the temperature of inward wind 30°C, the pressure of inward wind 0.5 bar, the temperature of inward wind 30°C, CYL: 3 bar, CAP1: 1.0 bar, peristaltic pump 6%, rotating rate of the turnplate 175 rpm, and then ethylcellulose, stearic acid and polyethylene glycol-6000 in 95% ethanol were sprayed to the material in the machine. After coating, drying was conducted at 50 °C, and then the materials were discharged.

| | | |
|---|---|---|
| c. | Atorvastatin calcium | 5g (calculated by the free acid) |
| | Blank pill core | 30g |
| | 7% PVP solution (90% ethanol as solvent) | 30g |

Preparing process: Passing atorvastatin calcium through a sieve of 120 mesh, weighing atorvastatin calcium as shown in the formulation, and then atorvastatin calcium was poured into a feeding hopper. Switching on granulating and coating machine, with the pressure of inward wind 0.5 bar, the temperature of inward wind 30°C, CYL: 3 bar, CAP1: 0.8 bar. Pouring the blank pill, and then granulating under the condition of feeding rate 4 rpm, peristaltic pump 12%, rotating rate of the turnplate 120 rpm, and spraying with 7% PVP solution (90% ethanol as solvent). After granulating, the resultant granules were dried at 45 °C, and then were discharged.
d. The pellets prepared in b and c were then filled into hard capsules by using a capsule filling machine, with 300 mg of acipimox and 5 mg of atorvastatin calcium (calculated by the free acid) contained in every two capsules.

### Example 7

| | | |
|---|---|---|
| a. | Acipimox | 400g |
| | Mannitol | 10g |
| | Lactose | 40g |
| | Microcrystal cellulose | 20g |
| | 6% PVP in 95% ethanol | 120g |
| | Magnesium stearate | 2g |

Preparing process: Passing acipimox through a sieve of 100 mesh, and passing mannitol, lactose, and microcrystal cellulose through a sieve of 80 mesh, weighing them as shown in the formulation and mixing them uniformly, and then appropriate amount of 6% PVP in 95% ethanol was added into the mixture for granulating. After granulating, drying the granules at 60°C, and passing the granules through a sieve of 16 mesh, and then magnesium stearate with the weight shown in the formulation was mixed into the dry granules.

| | | |
|---|---|---|
| b. | Atorvastatin calcium | 5g (calculated by the free acid) |
| | pregelatinized starch | 50g |
| | Mannitol | 50g |
| | Lactose | 40g |
| | 6% PVP in 95% ethanol | 100g |
| | Micronized silica gel | 5g |

Preparing process: Passing atorvastatin calcium through a sieve of 100 mesh, and passing pregelatinized starch, mannitol and lactose through a sieve of 80 mesh, weighing them as shown in the formulation and mixing them uniformly, and then an appropriate amount of 6% PVP in 95% ethanol solution was added into the mixture for granulating. After granulating, drying the granules at 60°C, and passing the granules through a sieve of 16 mesh, and then magnesium stearate with the weight shown in the formulation was mixed into the dry granules.
c. The resultant granules in a and b were compressed into double layer tablets by using double punches compression machine, with 400mg of acipimox and 5mg of atorvastatin calcium (calculated by the free acid) contained in each tablet.

### Example 8

| | | |
|---|---|---|
| a. | Acipimox | 400g |
| | Lactose | 30g |
| | Hydroxypropyl methylcellulose-15M | 20g |
| | 8% PVP in 95% ethanol | 150g |
| | Glyceryl behenate | 2g |

Preparing process: Passing acipimox through a sieve of 100 mesh, and passing lactose and hydroxypropyl methylcellulose-15M through a sieve of 80 mesh, weighing them as shown in the formulation and mixing them uniformly, and then appropriate amount of 8% PVP in 95% ethanol was added into the mixture for granulating. After granulating, drying the granules at 60 °C, and then passing the granules through a sieve of 16 mesh, glyceryl behenate with the weight shown in the formulation was then mixed into the dry granules.

| | | |
|---|---|---|
| b. | Atorvastatin calcium | 10g (calculated by the free acid) |
| | Hydroxypropyl cellulose | 25g |
| | Dextrine | 20g |
| | 6% PVP in 95% ethanol | 50g |
| | Talc powder | 2g |

Preparing process: Passing atorvastatin calcium through a sieve of 100 mesh, and passing hydroxypropyl cellulose and dextrine through a sieve of 80 mesh, weighing them as shown in the formulation and mixing them uniformly, and then an appropriate amount of 6% PVP in 95% ethanol solution was added into the mixture for granulating. After granulating, drying the granules at 60°C, and then passing the granules through a sieve of 16 mesh, magnesium stearate with the weight shown in the formulation was then mixed into the dry granules.
c. The resultant granules in a and b were compressed into double layer tablets by using double punches compression machine, with 400mg of acipimox and 10mg of atorvastatin calcium (calculated by the free acid) contained in each tablet.

### Example 9: Screening the formulation of acipimox in combination with atorvastatin calcium for treating hyperlipemia in rats

The objective of the present experiment is to determine the formulation of acipimox in combination with atorvastatin calcium with low toxicity and high activity, which is also convenient for administration, by means of screening. Hyperlipemia rat model was induced by feeding rats with high lipid containing feed. The model rats were continuously administered by gavage with acipimox (100 to 400 mg/kg) and (or) atorvastatin calcium (2.5 to 30 mg/kg, calculated by the free acid, the dosages described below were all calculated by atorvastatin free acid) for 14 days. The results showed that the formulation of acipimox in combination with atorvastatin calcium had remarkable therapeutic effect on hyperlipemia in rats induced by high lipid containing feed, and the lipid decreasing effect was related to the dosages of the two drugs. The formulations of acipimox 200 mg/kg in combination with atorvastatin calcium 10 mg/kg, acipimox 200 mg/kg in combination with atorvastatin calcium 20 mg/kg, and acipimox 300 mg/kg in combination with atorvastatin calcium 10 mg/kg were shown to have remarkable effect, in the aspect of decreasing the level of serum total cholesterol, triglyceride, low density lipoprotein-cholesterol in rats, and increasing the level of high density lipoprotein-cholesterol. These formulations were shown to make synergistic effects, among which the formulation of acipimox 300 mg/kg in combination with atorvastatin calcium 10 mg/kg had the most remarkable effect. Thus, the formulation of acipimox 300 mg/kg and atorvastatin calcium 10 mg/kg is most preferred. In the dosage range of the present experiment, the formulations were not shown to have any remarkable effect on the activity of seroenzyme which is related to the toxicities on the liver and muscles.

### 1. Experimental objective

The objective of the present experiment is to determine the formulation of acipimox in combination with atorvastatin calcium by screening, in order to obtain a formulation with low toxicity, extensive and enhanced effect and is convenient for use.

### 2. Test drugs

### 2.1 Nicotinic acid derivatives

Name of the drug: acipimox
Batch No. 0207002
Purity: >99.7%
Manufacturer: LuNan Pharmaceutical Company Limited

Preparing method: Acipimox was mixed uniformly with 1% carboxymethylcellulose sodium (CMC) just before use, in order to formulate the concentrations desired for the test.

### 2.2 statins

Name of the drug: atorvastatin calcium
Cat. No. 031002
Purity: >99.0%
Manufacturer: ShanDong Newtime Pharmaceuticals Co., Ltd.
Preparing method: atorvastatin calcium was mixed uniformly with 1% CMC just before use, in order to formulate the concentrations desired for the test.

### 3. Animals

### 3.1 Lines and Source

Wistar rats, bred by the Medical Experimental Animal Centre of Chinese Academy of Military Medical Sciences, certificate No. D01-3039.

### 3.2 Body weight and Gender

Nine to ten weeks old, body weight 180-220g, male

### 3.3 Feeding Conditions

The animal laboratory was ventilated regularly, well lighted, and kept at the room temperature. Five animals were kept in each cage, fed with expanded feed specially formulated for rats by the experimental animal centre of our academy, and were given tap water *ad libitum.* The certificate number of the animals' experimental condition is D01-2051. Before the experiment, the feeding, activity and dejection of the animals were observed for 1 week, and healthy animals were selected for the experiment.

### 4. The Establishment of Hyperlipemia Rat Model^{[1]}

Hyperlipemia rat model was established by inducing hyperlipemia with high lipid containing feed. The formulation of high lipid containing feed was as follows: basic feed 86.3%, cholesterol 3%, lard 10%, methylthiouracil 0.2% and porcine bile salt 0.5%, and were allowed to be mixed uniformly. The rats were continuously fed for 2 weeks. During the administration of drugs, high lipid containing feed was administered every other day.

### 5. Effects of Acipimox and Atorvastatin Calcium on the Level of Blood Lipid in Normal Rats 5.1 The Basis for Dosage Setting

The clinically used dosage of acipimox is 250mg per time (The above mentioned dosage is 4.2 mg/kg as calculated on the human body weight of 60 kg.), twice or thrice per day, with the daily dosage not more than 1200 mg^{[2]}. By calculating in accordance with the principle of dosage equivalent per body surface area, the above mentioned generally used dosage for human was converted into the dosage for rats of about 50 mg/kg/day. By referring to those had been reported^{[3]}, the dosages of acipimox in the present experiment were set as 100, 200, 300 and 400 mg/kg.

The clinically used dosage of atorvastatin calcium is 10mg per time (The above mentioned dosage is 0.17 mg/kg as calculated on the human body weight of 60 kg.), once per day, with the daily dosage not more than 80 mg^{[4]}. By calculating in accordance with the principle of dosage equivalent per body surface area, the above mentioned generally used dosage for human was converted into the dosage for rats of about 0.85 mg/kg/day. By referring to those had been reported^{[5-8]}, the dosages of atorvastatin calcium in the present experiment were set as 2.5, 5, 10, 20, and 30 mg/kg.

### 5.2 Grouping

Depending on the above mentioned dosage setting, and by following the balance principle of serum total cholesterol level, the normal animals were randomly grouped into: (1) normal control group; (2) group of acipimox 100 mg/kg; (3) group of acipimox 200 mg/kg; (4) group of acipimox 300 mg/kg; (5) group of acipimox 400 mgkg; (6) group of atorvastatin calcium 2.5 mg/kg; (7) group of atorvastatin calcium 5 mg/kg; (8) group of atorvastatin calcium 10 mg/kg; (9) group of atorvastatin calcium 20 mg/kg; (10) group of atorvastatin calcium 30 mg/kg; (11) group of the formulation of acipimox 200 mg/kg in combination with atorvastatin calcium 5 mg/kg; (12) group of the formulation of acipimox 200 mg/kg in combination with atorvastatin calcium 10 mg/kg; (13) group of the formulation of acipimox 200 mg/kg in combination with atorvastatin calcium 20 mg/kg; (14) group of the formulation of acipimox 300 mg/kg in combination with atorvastatin calcium 5 mg/kg; (15) group of the formulation of acipimox 300 mg/kg in combination with atorvastatin calcium 10 mg/kg; (16) group of the formulation of acipimox 300 mg/kg in combination with atorvastatin calcium 20 mg/kg; (17) group of the formulation of acipimox 400 mg/kg in combination with atorvastatin calcium 5 mg/kg; (18) group of the formulation of acipimox 400 mg/kg in combination with atorvastatin calcium 10 mg/kg. Each group included 10 rats.

### 5.3 Administration

The administering route intended for the clinical application is oral administration, and thus gavage administration was used in the present experiment for continuous 4 days. Gavage administration was conducted on the rats after feeding, once per day. The dosing volume is 0.3 ml/100 g of body weight.

### 5.4 Measurement

The serum bio-chemical index includes total cholesterol (TC), alanine transaminase (ALT), creatine kinase (CK), triglyceride (TG), low density lipoprotein-cholesterol (LDL-C), high density lipoprotein-cholesterol (LDL-C). The reagents for measuring alanine transaminase (ALT) and creatine kinase (CK) were produced by Beijing Zhongsheng High-Tech Bioengineering Company, and SABA/18 Automatic Biochemical Analyzer was used for measurement. Other reagents were produced by Roche Inc., Japan, and Hitachi 7020 Automatic Biochemical Analyzer was used for measurement. The measuring methods are based on the specifications of the reagents, and the rats were fasted for 16 hours before collecting blood.

### 6. Effects of Acipimox and Atorvastatin Calcium on the Blood Lipid Level in the Rats with Hyperlipemia

### 6.1. The Basis for Dosage setting

The basis for dosage setting is the same as the experiments conducted on normal rats as described in section 5.1.

### 6.2. Grouping

Depending on the above mentioned dosage setting, and by following the balance principle of serum total cholesterol level, the model animals were randomly grouped into: (1) normal control group; (2) model control group; (3) group of acipimox 100 mg/kg; (4) group of acipimox 200 mg/kg; (5) group of acipimox 300 mg/kg; (6) group of acipimox 400 mgkg; (7) group of atorvastatin calcium 2.5 mg/kg; (8) group of atorvastatin calcium 5 mg/kg; (9) group of atorvastatin calcium 10 mg/kg; (10) group of atorvastatin calcium 20 mg/kg; (11) group of atorvastatin calcium 30 mg/kg; (12) group of the formulation of acipimox 200 mg/kg in combination with atorvastatin calcium 5 mg/kg; (13) group of the formulation of acipimox 200 mg/kg in combination with atorvastatin calcium 10 mg/kg; (14) group of the formulation of acipimox 200 mg/kg in combination with atorvastatin calcium 20 mg/kg; (15) group of the formulation of acipimox 300 mg/kg in combination with atorvastatin calcium 5 mg/kg; (16) group of the formulation of acipimox 300 mg/kg in combination with atorvastatin calcium 10 mg/kg; (17) group of the formulation of acipimox 300 mg/kg in combination with atorvastatin calcium 20 mg/kg; (18) group of the formulation of acipimox 400 mg/kg in combination with atorvastatin calcium 5 mg/kg; (19) group of the formulation of acipimox 400 mg/kg in combination with atorvastatin calcium 10 mg/kg. Each group included 10 rats.

### 6.3 Administration

The administering route intended for the clinical application is oral administration, and thus gavage administration was used in the present experiment for continuous 14 days. Gavage administration was conducted on the rats after feeding, once per day. The dosing volume is 0.3 ml/100 g of body weight.

### 6.4 Measurement

The measurement is the same as the experiment conducted on normal rats as described in section 5.4.

### 7. The test for the comparison of the Different Formulations of Acipimox in combination with Atorvastatin Calcium

### 7.1 Grouping

The dosage of the formulation of acipimox in combination with atorvastatin calcium was determined from the groups of acipimox and atorvastatin calcium which had relatively remarkable therapeutic effect: (1) normal control group; (2) model control group; (3) group of acipimox 200 mg/kg; (4) group of acipimox 300 mg/kg; (5) group of atorvastatin calcium 10 mg/kg; (6) group of atorvastatin calcium 20 mg/kg; (7) group of the formulation of acipimox 200 mg/kg in combination with atorvastatin calcium 10 mg/kg; (8) group of the formulation of acipimox 200 mg/kg in combination with atorvastatin calcium 20 mg/kg; (9) group of the formulation of acipimox 300 mg/kg in combination with atorvastatin calcium 10 mg/kg. The level of serum total cholesterol level was respectively measured before grouping, and then randomly grouping by following the balance principle. Each group included 10 rats.

### 7.2 Administration and Measurement

The same as those described in sections 6.3 and 5.4.

### 8. Experimental Results

### 8.1 Effects of Acipimox and Atorvastatin Calcium on the Blood Lipid Level in the Normal Rats

After the administration to the normal rats for four days, the total cholesterol, serum triglyceride and low density lipoprotein-cholesterol in all the groups of acipimox, atorvastatin calcium and the formulation of acipimox in combination with atorvastatin calcium were shown to be decreased, and the level of high density lipoprotein-cholesterol was shown to be increased. The results were shown in Table 1.

**Table 1. Effects of acipimox, atorvastatin calcium and the formulation of acipimox in combination with atorvastatin calcium on the blood lipid in the normal rats**

| Groups | Total cholesterol in serum (mmol/L) | Triglyceride in serum (mmol/L) | Low density lipoprotein-cholesterol (mmol/L) | High density lipoprotein-cholesterol (mmol/L) |
|---|---|---|---|---|
| Normal control group | 1.74±0.19 | 0.73±0.25 | 0.34±0.10 | 0.95±0.19 |
| Acipimox 100 mg/kg | 1.60±0.35 | 0.71±0.27 | 0.32±0.12 | 0.97±0.16 |
| Acipimox 200 mg/kg | 1.55±0.28 | 0.69±0.33 | 0.26±0.11 | 0.98±0.23 |
| Acipimox 300 mg/kg | 1.51±0.26* | 0.63±0.28 | 0.29±0.11 | 1.15±0.26 |
| Acipimox 400 mg/kg | 1.49±0.28* | 0.57±0.36 | 0.24±0.14 | 1.18±0.13** |
| Atorvastatin calcium 2.5 mg/kg | 1.69±0.33 | 0.69±0.33 | 0.30±0.14 | 0.99±0.11 |
| Atorvastatin calcium 5 mg/kg | 1.64±0.30 | 0.67±0.28 | 0.25±0.16 | 0.96±0.11 |
| Atorvastatin calcium 10 mg/kg | 1.52±0.31 | 0.65±0.33 | 0.23±0.09* | 1.03±0.24 |
| Atorvastatin calcium 20 mg/kg | 1.46±0.24* | 0.55±0.16 | 0.21±0.09* | 1.05±0.13 |
| Atorvastatin calcium 30 mg/kg | 1.41±0.29* | 0.51±0.18* | 0.19±0.12* | 1.08±0.18 |
| Acipimox 200 + Atorvastatin calcium 5 mg/kg | 1.53±0.24 | 0.61±0.33 | 0.23±0.09* | 1.08±0.20 |
| Acipimox 200 + Atorvastatin calcium 10 mg/kg | 1.47±0.24* | 0.49±0.11* | 0.19±0.1** | 1.15±0.16* |
| Acipimox 200 + Atorvastatin calcium 20 mg/kg | 1.38±0.32** | 0.46±0.13** | 0.17±0.06*** | 1.19±0.16** |
| Acipimox 300 + Atorvastatin calcium 5 mg/kg | 1.46±0.25* | 0.63±0.22 | 0.27±0.06 | 0.99±0.22 |
| Acipimox 300 + Atorvastatin calcium 10 mg/kg | 1.44±0.26* | 0.50±0.06* | 0.18±0.12** | 1.20±0.15** |
| Acipimox 300 + Atorvastatin calcium 20 mg/kg | 1.45±0.27* | 0.50±0.17* | 0.20±0.09** | 1.09±0.24 |
| Acipimox 400 + Atorvastatin calcium 5 mg/kg | 1.44±0.31* | 0.52±0.12* | 0.25±0.10 | 1.03±0.17 |
| Acipimox 400 + Atorvastatin calcium 10 mg/kg | 1.41±0.36* | 0.51±0.14* | 0.24±0.08* | 1.11±0.21 |

| | | | | |
|---|---|---|---|---|
| Note: *P<0.05, **P<0.01 vs. normal control group. | | | | |

### 8.2 Effects of Acipimox and Atorvastatin Calcium on the Blood Lipid Level in the Model

### Rats

The rats were divided into groups after being administered high lipid containing feed for 14 days. The groups of the formulation of acipimox in combination with atorvastatin calcium were consisted of acipimox with dosage ranging from 100 to 400 mg/kg, and atorvastatin calcium with dosage ranging from 2.5 to 30 mg/kg. After administering for 14 days, the level of serum total cholesterol, triglyceride and low density lipoprotein-cholesterol in all the groups of acipimox and atorvastatin calcium were shown to be decreased to different degrees, and the level of high density lipoprotein-cholesterol was shown to be increased, in comparison with the model control group. The results were shown in Table 2.

As compared with the model control group, the level of serum total cholesterol, triglyceride and low density lipoprotein-cholesterol in the rats of the three groups of the formulation of acipimox 200 mg/kg in combination with atorvastatin calcium 10 mg/kg, the formulation of acipimox 200 mg/kg in combination with atorvastatin calcium 20 mg/kg, and the formulation of acipimox 300 mg/kg in combination with atorvastatin calcium 10 mg/kg were shown to be remarkably decreased, and the level of high density lipoprotein-cholesterol was shown to be remarkably increased. Furthermore, the above-mentioned effects were shown to be synergistic. The effect of the group of the formulation of acipimox 300 mg/kg in combination with atorvastatin calcium 10 mg/kg was shown to be the most remarkable.

**Table 2. Effects of acipimox, atorvastatin calcium and the formulation of acipimox in combination with atorvastatin calcium on the blood lipid in the model rats**

| Groups | Total cholesterol in serum (mmol/L) | Triglyceride in serum (mmol/L) | Low density lipoprotein-cholesterol (mmol/L) | High density lipoprotein-cholesterol (mmol/L) |
|---|---|---|---|---|
| Normal control group | 1.74±0.19 | 0.71±0.17 | 0.25±0.14 | 0.95±0.19 |
| Model control group | 13.73±2.80^{###} | 2.23±0.54^{###} | 9.26±1.21^{###} | 3.87±0.86^{###} |
| Acipimox 100 mg/kg | 13.18±2.31 | 1.98±0.42 | 8.44±1.06 | 3.95±0.63 |
| Acipimox 200 mg/kg | 10.82±3.05* | 1.78±0.55 | 8.23±0.40* | 4.35±0.55 |
| Acipimox 300 mg/kg | 10.73±2.36* | 1.69±0.51* | 7.79±0.89** | 4.56±0.50 |
| Acipimox 400 mg/kg | 11.18±2.47 | 1.72±0.54 | 7.65±0.40** | 4.70±0.58* |
| Atorvastatin calcium 2.5 mg/kg | 13.64±1.82 | 2.22±0.56 | 8.87±0.74 | 4.16±0.91 |
| Atorvastatin calcium 5 mg/kg | 12.45±2.67 | 1.93±0.52 | 8.17±0.80* | 4.21±0.59 |
| Atorvastatin calcium 10 mg/kg | 11.23±2.12* | 1.80±0.28* | 8.07±0.55* | 4.48±0.41 |
| Atorvastatin calcium 20 mg/kg | 10.27±2.67* | 1.72±0.47* | 7.78±0.26** | 4.52±0.32* |
| Atorvastatin calcium 30 mg^{/}kg | 9.91±2.65** | 1.78±0.55 | 7.98±0.60* | 4.59±0.27* |
| Acipimox 200 + Atorvastatin calcium 5 mg/kg | 7.73±2.10*** | 1.27±0.19** | 5.76±0.51*** | 5.48±0.52*** |
| Acipimox 200 + Atorvastatin calcium 10 mg/kg | 7.02±1.62*** | 1.19±0.15*** | 5.37±0.41*** | 5.97±0.30*** |
| Acipimox 200 + Atorvastatin calcium 20 mg/kg | 6.07±2.00*** | 1.17±0.21*** | 5.21±0.59*** | 6.30±0.33*** |
| Acipimox 300 + Atorvastatin calcium 5 mg/kg | 8.01±2.06*** | 1.23±0.23** | 5.15±0.70*** | 5.75±0.58*** |
| Acipimox 300 + Atorvastatin calcium 10 mg/kg | 7.08±2.01*** | 1.15±0.20*** | 4.94±0.70*** | 6.04±0.52*** |
| Acipimox 300 + Atorvastatin calcium 20 mg/kg | 6.93±2.59*** | 1.14±0.26*** | 4.80±0.48*** | 6.16±0.51*** |
| Acipimox 400 + Atorvastatin calcium 5 mg/kg | 7.95±1.88*** | 1.20±0.23*** | 5.19±0.87*** | 5.80±0.47*** |
| Acipimox 400 + Atorvastatin calcium 10 mg/kg | 7.27±2.55*** | 1.15±0.31*** | 5.17±0.76*** | 5.95±0.45*** |

| | | | | |
|---|---|---|---|---|
| Note: ###P<0.001 vs. normal control group; *P<0.05, **P<0.01, ***P<0.001 vs. model control group. | | | | |

### 8.3 Effects of Acipimox and Atorvastatin Calcium on the Activity of Seroenzyme in the Model Rats

In this experiment, by measuring the level of alanine transaminase and creatine kinase in the serum of hyperlipemia model rats after being administered for 14 days, the effects of acipimox, atorvastatin calcium and the combination thereof on the function of liver and skeletal muscle tissue in the model rats were evaluated. The results showed that in comparison with the model control group, the levels of alanine transaminase and creatine kinase in serum of model rats in acipimox groups at various dosages (100 to 400 mg/kg) were not remarkably different (P>0.05); the levels in serum of model rats of atorvastatin calcium groups at various dosages (2.5 to 30 mg/kg) were not remarkably different (P>0.05), either; the level of alanine transaminase and creatine kinase in serum of the rats in all the groups of acipimox in combination with atorvastatin calcium were not remarkably different (P>0.05), either. The results were shown in Table 3.

**Table 3. Effects of acipimox, atorvastatin calcium and the formulation of acipimox in combination with atorvastatin calcium on alanine transaminase and creatine kinase in serum of the model rats**

| Groups | Alanine transaminase (nmol.s⁻¹/L) | Creatine kinase (U/L) |
|---|---|---|
| Normal control group | 639.5±111.3 | 351.4±144.2 |
| Model control group | 667.9±109.9 | 373.6±112.4 |
| Acipimox 100 mg/kg | 640.3±117.6 | 361.4±106.4 |
| Acipimox 200 mg/kg | 645.4±124.9 | 354.4±110.9 |
| Acipimox 300 mg/kg | 655.5±110.5 | 380.33±120.1 |
| Acipimox 400 mg/kg | 642.8±114.2 | 377.3±103.4 |
| Atorvastatin calcium 2.5 mg/kg | 635.0±115.2 | 364.6±105.9 |
| Atorvastatin calcium 5 mg/kg | 674.5±132.9 | 367.7±132.6 |
| Atorvastatin calcium 10 mg/kg | 650.0±101.8 | 389.0±119.1 |
| Atorvastatin calcium 20 mg/kg | 640.6±95.6 | 378.8±109.3 |
| Atorvastatin calcium 30 mg/kg | 66.4±117.7 | 384.2±120.8 |
| Acipimox 200 + Atorvastatin calcium 5 mg/kg | 653.4±110.5 | 374.1±101.5 |
| Acipimox 200 + Atorvastatin calcium 10 mg/kg | 669.7±123.0 | 353.3±124.6 |
| Acipimox 200 + Atorvastatin calcium 20 mg/kg | 641.6±125.1 | 367.4±111.4 |
| Acipimox 300 + Atorvastatin calcium 5 mg/kg | 604.9±132.6 | 361.9±115.2 |
| Acipimox 300 + Atorvastatin calcium 10 mg/kg | 647.7±128.2 | 370.6±130.0 |
| Acipimox 300 + Atorvastatin calcium 20 mg/kg | 646.6±124.0 | 372.1±117.3 |
| Acipimox 400 + Atorvastatin calcium 5 mg/kg | 651.9±136.1 | 369.4±103.2 |
| Acipimox 400 + Atorvastatin calcium 10 mg/kg | 647.3±140.6 | 375.8±119.9 |

### 8.4 The Test for the comparison of the different Formulations of Acipimox in combination with Atorvastatin Calcium

After hyperlipemia model rats were continuously administered for 14 days, in comparison with either of Acipimox and Atorvastatin Calcium at the same dosage, all the groups of the formulations of acipimox in combination with atorvastatin calcium were shown to decrease more remarkably the level of total cholesterol, triglyceride and low density lipoprotein-cholesterol in serum of model rats, and more remarkably increase the level of high density lipoprotein-cholesterol. The results were shown in Table 4.

By comprehensive analysis, in comparison with model control group, the groups of the formulations of acipimox 200 mg/kg in combination with atorvastatin calcium 10 mg/kg. acipimox 200 mg/kg in combination with atorvastatin calcium 20 mg/kg, and acipimox 300 mg/kg in combination with atorvastatin calcium 10 mg/kg were shown to have more remarkable effect, and were shown to decrease more remarkably the level of total cholesterol, triglyceride, and low density lipoprotein-cholesterol in serum of rats, and were shown to increase more remarkably the level of high density lipoprotein-cholesterol. In comparison with either of acipimox and atorvastatin calcium at the same dosage, the levels of total cholesterol, triglyceride and low density lipoprotein-cholesterol in serum of the groups of the above three formulations of acipimox in combination with atorvastatin calcium were shown to be remarkably decreased, and the level of high density liproprotein-cholesterol was shown to be remarkably increased. The above-mentioned effects were shown to be synergistic in the above three groups. Among the three groups, the group of the formulation of acipimox 300 mg/kg in combination with atorvastatin calcium 10 mg/kg was shown to have the most remarkable effect. Therefore, the formulation of acipimox 300 mg/kg and atorvastatin calcium 10 mg/kg is preferred.

**Table 4: The comparison of the Effects of the Different Formulations of Acipimox in combination with Atorvastatin Calcium on hyperlipemia of rats**

| Groups | Total cholesterol in serum (mmol/L) | Triglyceride in serum (mmol/L) | Low density lipoprotein-cholesterol (mmoUL) | High density lipoprotein-cholesterol (mmol/L) |
|---|---|---|---|---|
| Normal control group | 2.09±0.96 | 0.78±0.18 | 0.30±0.14 | 1.26±0.50 |
| Model control group | 12.14±2.24^{###} | 2.14±0.50^{###} | 8.93±1.36^{###} | 3.18±1.27*** |
| Acipimox200mg/kg | 10.31=1.67 | 1.79±0.63 | 7.27±1.83* | 4.20±1.10 |
| Acipimox 300 mg/kg | 8.69±1.88** | 1.68±0.56 | 6.96±1.23** | 4.54±0.52* |
| Atorvastatin calcium 10 mg/kg | 10.20±1.62* | 1.56±0.62* | 7.30±1.88* | 4.46±1.3* |
| Atorvastatin calcium 20 mg/kg | 9.65±2.22* | 1.47±0.56* | 6.80±1.66* | 4.51±1.09* |
| Acipimox 200 + Atorvastatin calcium 10 mg/kg | 7.43±1.67***^{,",@@} | 0.99±0.22***^{,"@} | 4.91±1.26***^{,",@@} | 5.95±1.07***^{,",@} |
| Acipimox 200 + Atorvastatin calcium 20 mg/kg | 6.34±1.81***'"^{&&&} | 0.82±0.26***'"^{&&} | 4.25±1.63***^{,",&&} | 6.07±1.32***^{,",&} |
| Acipimox 300 + Atorvastatin calcium 10 mg/kg | 4.82±1.03***^{,^^^,@@@} | 0.73=0.23***^{,^^^,@@} | 3.95±1.28***^{,^^^,@@@} | 6.45±1.07***^{,^^^,@@} |

| | | | | |
|---|---|---|---|---|
| Note: ###P<0.01, #P<0.05 vs. normal control group; *P<0.05, **P<0.01, ***P<0.001 vs. model control group; !!!P<0.001, !!P<0.01 vs. the group of acipimox 200 mg/kg; @P<0.05, @@P<0.01, @@@P<0.001 vs. the group of atorvastatin calcium 10 mg/kg; ^^^P<0.001 vs. the group of acipimox 300 mg/kg; &&&P<0.001. &P<0.05 vs. the group of atorvastatin calcium 20 mg/kg. | | | | |

### 9. Conclusions

The combination of acipimox with atorvastatin calcium had remarkable therapeutic effect on hyperlipemia in rat induced by high lipid containing feed, and the lipid decreasing effect was related to the dosages of the two drugs. The three groups of the formulations of acipimox 200 mg/kg in combination with atorvastatin calcium 10 mg/kg, acipimox 200 mg/kg in combination with atorvastatin calcium 20 mg/kg, and acipimox 300 mg/kg in combination with atorvastatin calcium 10 mg/kg were shown to have remarkable effect, in particularly remarkably decreasing the levels of total cholesterol, triglyceride, low density lipoprotein-cholesterol in serum of rats, and remarkably increasing the level of high density lipoprotein-cholesterol. Furthermore, the above-mentioned effects were shown to be synergistic. Among the groups, the group of the formulation of acipimox 300 mg/kg in combination with atorvastatin calcium 10 mg/kg was shown to have the most remarkable effect, and thus the formulation of acipimox 300 mg/kg in combination with atorvastatin calcium 10 mg/kg is preferred. In the range of the dosage used in the present experiment, the groups of the formulations of acipimox in combination with atorvastatin calcium were shown to have no remarkable effect on the activity of seroenzyme, preliminarily indicating no remarkable toxic effect on the liver and striated muscle. Acipimox alone is conventionally administered thrice per day, however the present experiment demonstrates that the administration of acipimox once per day still has remarkable lipid decreasing effect and small toxicity, which provides reliable experimental support for the administering scheme of the formulation of acipimox in combination with atorvastatin calcium as once per day. This administering scheme will definitely make the medicament more convenient to the patients, so as to increase the compliance of the patients.

### Example 10

### Lipid decreasing effect of the combination of acipimox and atorvastatin calcium in comparison with those of the combination of acipimox and pravastatin, as well as the combination of acipimox and lovastatin

In the experiment, we compared the formulation of acipimox in combination with atorvastatin calcium screened in Example 9 with the compositions disclosed in the above mentioned patents, and surprisingly found that the combination of acipimox and atorvastatin calcium was not only shown to be remarkably synergistic in decreasing serum total cholesterol, serum triglyceride and low density lipoprotein-cholesterol, but also was shown to be remarkably different from the effect of the combination of acipimox and pravastatin, as well as that of the combination of acipimox and lovastatin. That is to say that the combination of acipimox and atorvastatin calcium exhibited more remarkable effects for decreasing lipid and increasing high density liproprotein-cholesterol. This is based on a large amount of *in vivo* experiments.

### 10.1 The test drugs, animals and the establishment of hyperlipemia rat model were same as those described in Example 9.

### 10.2 Grouping

By following the balance principle of serum total cholesterol level, the model rats were randomly divided into the following groups:
normal control group;
model control group;
group of acipimox 300 mg/kg and pravastatin 20 mg/kg;
group of acipimox 300 mg/kg and lovastatin 10 mg/kg;
group of acipimox 200 mg/kg and atorvastatin calcium 5 mg/kg;
group of acipimox 200 mg/kg and atorvastatin calcium 10 mg/kg;
group of acipimox 200 mg/kg and atorvastatin calcium 20 mg/kg;
group of acipimox 300 mg/kg and atorvastatin calcium 5 mg/kg;
group of acipimox 300 mg/kg and atorvastatin calcium 10 mg/kg;
group of acipimox 300 mg/kg and atorvastatin calcium 20 mg/kg;
group of acipimox 400 mg/kg and atorvastatin calcium 5 mg/kg;
group of acipimox 400 mg/kg and atorvastatin calcium 10 mg/kg.

### 10.3 Administration

The administration method was the same as described in section 6.3 of Example 9.

### 10.4 Measurement

Total cholesterol (TC), triglyceride (TG), low density lipoprotein-cholesterol (LDL-C), and high density lipoprotein-cholesterol (HDL-L) in serum were measured.

### 10.5 Experimental Results

After fed with high lipid containing feed for 14 days, the rats were divided into groups and administered with drugs. After being administered for 14 days, all of the groups of acipimox in combination with atorvastatin calcium at various dosages were shown to be remarkably different from the group of acipimox 300 mg/kg in combination with pravastatin 20 mg/kg and the group of acipimox 300 mg/kg in combination with lovastatin 10 mg/kg in decreasing serum total cholesterol, serum triglyceride and low density lipoprotein-cholesterol, as well as in increasing high density lipoprotein-cholesterol. The experimental results fully demonstrated that the combination of acipimox and atorvastatin calcium had unexpected effect in decreasing the blood lipid. The combination of acipimox and atorvastatin calcium achieved not only remarkable synergistic effect, but also exhibited the advantageous effects over the combinations of acipimox and lovastatin as welll as acipimox and pravastatin as disclosed in the prior art. The detailed results were shown in Table 5.

**Table 5. Lipid decreasing effect of the combination of acipimox and atorvastatin calcium in comparison with those of the combinations of acipimox and pravastatin, as well as acipimox and lovastatin**

| Groups | Total cholesterol in the serum (mmol/L) | Triglyceride in the serum (mmol/L) | Low density lipoprotein-cholesterol (mmol/L) | High density lipoprotein-cholesterol (mmol/L) |
|---|---|---|---|---|
| Normal control group | 1.74±0.19 | 0.71±0.17 | 0.25±0.14 | 0.95±0.19 |
| Model control group | 13.73±2.80^{###} | 2.23±0.54^{###} | 9.26±1.21^{###} | 3.87±0.86^{###} |
| Acipimox 200 + Atorvastatin calcium 5 mg/kg | 7.73±2.10**** | 1.27±0.19*** | 5.76±0.51***** | 5.48±0.52*** |
| Acipimox 200 + Atorvastatin calcium 10 mg/kg | 7.02±1.62**** | 1.19±0.15***** | 5.37±0.41***** | 5.97±0.30***** |
| Acipimox 200 + Atorvastatin calcium 20 mg/kg | 6.07±2.00***** | 1.17+0.21***** | 5.21±0.59***** | 6.30±0.33***** |
| Acipimox 300 + Atorvastatin calcium 5 mg/kg | 8.01±2.06*** | 1.23±0.23*** | 5.15±0.70***** | 5.75±0.58**** |
| Acipimox 300 + Atorvastatin calcium 10 mg/kg | 7.08±2.01**** | 1.15±0.20***** | 4.94±0.70***** | 6.04±0.52***** |
| Acipimox 300 + Atorvastatin calcium 20 mg/kg | 6.93±2.59***** | 1.14±0.26***** | 4.80±0.48***** | 6.16±0.51***** |
| Acipimox 400 + Atorvastatin calcium 5 mg/kg | 7.95±1.88**** | 1.20±0.23**** | 5.19±0.87***** | 5.80±0.47***** |
| Acipimox 400 + Atorvastatin calcium 10 mg/kg | 7.27±2.55***** | 1.15±0.31***** | 5.17±0.76***** | 5.95±0.45***** |
| Acipimox 300 + Pravastatin 20 mg/kg | 9.75±2.37** | 1.53±0.25** | 6.83±0.51*** | 4.92±0.63* |
| Acipimox 300 + Lovastatin 10 mg/kg | 9.91±1.67** | 1.59±0.26** | 6.69±0.92*** | 5.11±0.58** |

| | | | | |
|---|---|---|---|---|
| Note: ###P<0.001 vs. normal control group; **P<0.01, ***P<0.001 vs. model control group; *P<0.05, **P<0.01 vs. groups of acipimox 300 mg/kg in combination with pravastatin 20 mg/kg and acipimox, 300 mg/kg in combination with lovastatin 10 mg/kg. | | | | |

### References

[1] Xu Shu-Yun, Bian Ru-Lian, Chen Xiu. Pharmacological Experimental methodology (3rd Ed.), People's Medical Publishing House, January 2002, 1201-1202
[2] The specification of OLBETAM (acipimox) (Pharmacia, New Zealand)
[3] Scan J Clin Lab Invest, 1990, 50(2):203-208
[4] Guideline for using atorvastatin calcium tablets, http://www.sda.gov.cn, Web site of State Food and Drug Administration
[5] Clin. Chim. Acta. 2004, 339(1-2):189-194
[6] Int. J. Cardiol. 2003, 91(1):59-69
[7] Metabolism 2003, 52(5):609-615
[8] Biochim. Biophys. Acta. 2002, 1580(2-3):161-170

## Claims

1. A composition comprising:
a) the first active component: acipimox; and
b) the second active component: atorvastatin, or the pharmaceutically acceptable salt, ester or solvate thereof,
wherein the weight ratio of the first active component to the second active component is (10-80):1.

2. The composition according to claim 1, **characterized in that** said pharmaceutically acceptable salt of atorvastatin is selected from sodium salt, calcium salt, potassium salt, magnesium salt, zinc salt or iron salt.

3. The composition according to claim 1, **characterized in that** said pharmaceutically acceptable ester of atorvastatin is selected from an ester formed with aliphatic alcohol, aromatic alcohol or heterocylic alcohol.

4. The composition according to claim 1 or 2, **characterized in that** said second active component is calcium salt of atorvastatin.

5. The composition according to claim 1, **characterized in that** the weight ratio of the first active component to the second active component is (10-30):1.

6. The composition according to claim 5, **characterized in that** the weight ratio of the first active component to the second active component is 30:1.

7. The composition according to any one of claims 1 to 6, **characterized in that** the composition is in the form of tablets, capsules, granules, pills or dropping pills.

8. Use of a composition of any one of claims 1 to 7 for the manufacture of a medicament useful for treating hyperlipaemia.

## Patentansprüche

1. Zusammensetzung umfassend:
a) die erste aktive Komponente Acipimox und
b) die zweite aktive Komponente Atorvastatin oder das/den pharmazeutisch geeignete/n Salz, Ester oder Solvat davon,
wobei das Gewichtsverhältnis der ersten aktiven Komponente zur zweiten aktiven Komponente (10-80):1 ist.

2. Zusammensetzung nach Anspruch 1, **dadurch gekennzeichnet, daß** das pharmazeutisch geeignete Salz des Atorvastatin aus Natriumsalz, Calciumsalz, Kaliumsalz, Magnesiumsalz, Zinksalz oder Eisensalz ausgewählt ist.

3. Zusammensetzung nach Anspruch 1, **dadurch gekennzeichnet, daß** der pharmazeutisch geeignete Ester des Atorvastatin aus einem mit aliphatischem Alkohol gebildeten Ester, aromatischen Ester oder heterozyklischen Ester ausgewählt ist.

4. Zusammensetzung nach Anspruch 1 oder 2, **dadurch gekennzeichnet, daß** die zweite aktive Komponente Calciumsalz des Atorvastatin ist.

5. Zusammensetzung nach Anspruch 1, **dadurch gekennzeichnet, daß** das Gewichtsverhältnis der ersten aktiven Komponente zur zweiten aktiven Komponente (10-30):1 ist.

6. Zusammensetzung nach Anspruch 5, **dadurch gekennzeichnet, daß** das Gewichtsverhältnis der ersten aktiven Komponente zur zweiten aktiven Komponente 30:1 ist.

7. Zusammensetzung nach einem der Ansprüche 1 bis 6, **dadurch gekennzeichnet, daß** die Zusammensetzung in der Form von Tabletten, Kapseln, Granulat, Pillen oder Tropfdragées vorliegt.

8. Verwendung der Zusammensetzung nach einem der Ansprüche 1 bis 7 zur Herstellung eines zur Behandlung von Hyperlipämie verwendbaren Medikaments.

## Revendications

1. Composition comprenant :
a) le premier composant actif ; acipimox ; et
b) le second composant actif : atorvastatine, ou son sel, ester ou solvate pharmaceutiquement acceptable,
où le rapport en poids du premier composant actif au second composant actif est (10-80):1.

2. Composition selon la revendication 1, **caractérisée en ce que** ledit sel pharmaceutiquement acceptable de l'atorvastatine est choisi parmi le sel de sodium, le sel de calcium, le sel de potassium, le sel de magnésium, le sel de zinc et le sel de fer.

3. Composition selon la revendication 1, **caractérisée en ce que** ledit ester pharmaceutiquement acceptable de l'atorvastatine est choisi parmi un ester formé avec un alcool aliphatique, un alcool aromatique ou un alcool hétérocyclique.

4. Composition selon la revendication 1 ou 2, **caractérisée en ce que** ledit second composant actif est le sel de calcium de l'atorvastatine.

5. Composition selon la revendication 1, **caractérisée en ce que** le rapport en poids du premier composant actif au second composant actif est (10-30):1.

6. Composition selon la revendication 5, **caractérisée en ce que** le rapport en poids du premier composant actif au second composant actif est 30:1.

7. Composition selon l'une quelconque des revendications 1 à 6, **caractérisée en ce que** la composition est sous forme de comprimés, capsules, granules, pilules ou pilules à chute (« dropping pills »).

8. Utilisation d'une composition selon l'une quelconque des revendications 1 à 7 pour la fabrication d'un médicament utile pour traiter l'hyperlipémie.
